# EUROPEAN PATENT APPLICATION

(11) **EP 4 775 214 A1**
(43) Date of publication of application: **15.07.2026**
(21) Application number: 26151625.6
(22) Date of filing: 13.01.2026
(51) Int. Cl.: A61K 36/28, A23L 33/105, A61K 47/00, A61P 1/16, A61K 31/685, A61K 31/718, A61K 31/722

(54) **NUTRACEUTICAL OR PHARMACEUTICAL COMPOSITION COMPRISING AN EXTRACT OF SILYBUM MARIANUM FOR USE IN THE TREATMENT AND/OR PREVENTION OF HEPATO-BILIARY CONDITIONS OR DISEASES**

(30) Priority: 13.01.2025 IT 202500000408
(71) Applicant: Neilos S.r.l., 80063 Piano di Sorrento (NA) (IT)
(72) Inventor: DI MAIO, Umberto, 80065 Sant' Agnello NA (IT)
(74) Representative: Di Giovine, Paolo

(57) **Abstract**

The present invention relates to a composition of substances, preferably obtained from natural sources, effective in the prevention and/or treatment of hepatobiliary conditions and diseases. The composition of the invention comprises an extract of *Silybum marianum,* phosphatidylserine, phosphatidylcholine, starch and chitosan. This formulation confers on the composition gastro-resistance properties, increased permeability through membranes and tight junctions, mucoadhesion, controlled release and high bioavailability of the active ingredient silymarin. The composition of the invention is prepared in the form of a solid, semisolid or liquid pharmaceutical dosage form, preferably for oral administration.

## Description

The present inventions refers to a nutraceutical or pharmaceutical composition of substances effective in the prevention and/or treatment of hepatobiliary conditions and pathologies in both humans and animals.

### HEPATOBILIARY DISEASES

Hepatobiliary diseases include diseases affecting the liver and bile ducts.

In the case of the bile ducts, the diseases mainly concern problems related to bile and its flow, which therefore cause bile retention.

Liver diseases (hepatopathies) are a class of disorders that affect one of the most important organs in our body, the liver.

The liver is the organ responsible for detoxification and is involved in the metabolism of lipids and sugars. In general, diseases affecting the liver can be infectious or non-infectious in origin.

Infectious liver diseases are mainly caused by specific viral pathogens such as hepatitis viruses, of which there are five, identified by letters of the alphabet, and other viruses such as cytomegalovirus, Epstein-Barr and herpes simplex virus, which do not have a specific tropism for the liver but which, under certain conditions, such as significant immunosuppression, can have significant consequences for the liver.

On the other hand, non-infectious liver diseases can be caused by various factors, such as exposure to particular substances or agents, such as drugs, alcohol abuse, pesticides, pollution, cigarette smoke, and are also associated with an unhealthy lifestyle that includes a diet rich in lipids with little or no physical activity. In recent years, due to the increased consumption of high-fat foods and continuous exposure to pollutants and toxins, the incidence of non-infectious liver diseases has increased exponentially, becoming one of the leading causes of hospitalisation and death, not to mention the significant impact on the healthcare system.

It can undoubtedly be said that hepatic steatosis, or fatty liver, is the most common cause of chronic liver disease. It affects about 30-40% of men and 15-20% of women.

Steatosis begins with a high accumulation of lipids in the liver and may or may not be associated with excessive alcohol consumption, which is why it is differentiated into NAFLD (*Non-Alcoholic Fatty Liver Disease*) and AFLD (*Alcoholic Fatty Liver Disease*) respectively. The specific cause of this condition has not yet been clarified, but genetic factors and lifestyle are certainly involved.

NAFLD is a reversible stage of the disease, and a change in diet and increased physical activity may be sufficient to restore physiological levels. The problem is that in almost all cases, NAFLD is asymptomatic and, as a result, appropriate measures are not taken to counteract the progression of the disease.

In fact, approximately 30-40% of patients suffering from NAFLD will develop non-alcoholic steatohepatitis, and of these, in 40-50% of cases, the disease will progress and liver fibrosis will develop. The disease can progress further, causing total loss of liver function or even hepatocellular carcinoma. In addition, there is growing scientific evidence that NAFLD is closely related to metabolic syndrome, insulin resistance, atherosclerosis, obesity, dyslipidaemia and hypertension. It has been estimated that NAFLD will become the leading cause of liver transplantation by 2030.

As mentioned, chronic alcohol consumption is another cause of hepatic steatosis and, in particular, consumption of more than 30 grams/day of ethanol is directly related to an increased risk of chronic liver damage and cirrhosis.

As mentioned above, there is currently no actual pharmacological treatment for fatty liver and hepatic steatosis.

In addition, according to European guidelines, pharmacological intervention should only be used in patients with stage II liver fibrosis or in patients at risk of disease progression, such as those suffering from metabolic syndrome, diabetics, people with high transaminase levels and high necro-inflammatory activity. The first approach to be taken is a change in lifestyle, in favour of a healthy diet and exercise. A second approach consists of administering substances with antioxidant activity. In fact, European, American and Japanese guidelines recommend the use of vitamin E. Another drug used is pioglitazone, a substance with PPAR-γ receptor agonist activity, used in the treatment of type 2 diabetes mellitus.

Pioglitazone is recommended by both American and Japanese guidelines, while European guidelines do not recommend its use, but it may be considered.

Other traditional drugs with hypoglycaemic or lipid-lowering activity, such as statins, metformin and pentoxifylline, have been tested and evaluated, but none have had any particular success and the use of these agents is not currently recommended by any guidelines. As for the treatment of infectious hepatitis, which is now increasingly rare in industrialised countries due to widespread vaccination against hepatitis A and hepatitis B viruses, in cases of acute disease, supportive therapy is usually administered, as the symptoms are self-limiting and tend to resolve spontaneously. In cases of chronic hepatitis, there are classes of highly effective antiviral drugs, such as the recent class of HCV (hepatitis C virus) polymerase inhibitors represented by sofosbuvir, which is around 90% effective in eradicating the infection.

This has been a huge step forward, as previously, once HCV infection became chronic, it progressed inexorably towards cirrhosis, requiring a liver transplant or progression to hepatocellular carcinoma. However, despite its high efficacy, the use of this class of drugs is not without side effects, some of which are serious. For this reason, it is important to research new substances, combinations, formulations or new approaches that can complement drug therapies and promote the prevention and treatment of liver diseases.

### GLOSSARY

The terms used in this description are as generally understood by those skilled in the art, unless otherwise indicated.

The term 'extract', in the context of this description, refers to any product derived from a plant drug, including all products derived from mechanical processing (pulverisation, grinding, mixing and/or other methods) or extraction processes (solvent extraction, distillation and/or other specific methods) performed on a drug.

### DETAILED DESCRIPTION OF THE INVENTION

*Silybum marianum,* commonly known as milk thistle, is one of the most important medicinal plants, attributed with significant hepatoprotective properties, such that it is used in the treatment of liver diseases such as infectious hepatitis, cirrhosis, jaundice and toxin poisoning.

Milk thistle is also used in the treatment of extrahepatic diseases, as it is believed to have antidiabetic, cardioprotective, anti-inflammatory, anti-fibrotic, lipid-lowering, immunomodulatory and neuroprotective properties.

The active substances in the extract are commonly identified by the term silymarin, which refers to the mixture of flavonoids silibin (also called silibinin), isosilibin, silidianin and silicristin.

One of the underlying causes of various liver diseases, such as liver fibrosis, is the imbalance between the production of reactive oxygen species (ROS) and their neutralisation by endogenous antioxidants.

The situation described above is known as oxidative stress, which promotes and maintains pro-inflammatory stimulation. In various models, silibin has been shown to be a powerful antioxidant, particularly against hydroxyl and peroxyl anions. In addition, this substance has been shown to inhibit superoxide anion radicals and nitric oxide in isolated Kupffer cells. Milk thistle exerts its hepatoprotective activity not only by acting on this target, but also through other mechanisms. One of the main ones is its ability to stabilise membrane permeability by inhibiting lipid peroxidation. In addition, silymarin has been shown to increase endogenous glutathione levels.

Furthermore, silymarin has been shown to be effective in preventing damage induced by various toxicological agents such as drugs or chemicals such as carbon tetrachloride. This action is carried out by inhibiting hepatic NF-kB, resulting in a decrease in the production of TNF-α, INF-γ, IL-2 and IL-4.

Furthermore, milk thistle has also been shown in further studies to have anti-inflammatory and anti-fibrotic activity.

In a study conducted on isolated Kupffer cells, silymarin inhibited the formation of leukotriene B4 (LBT4), demonstrating the anti-inflammatory potential of milk thistle.

In addition, anti-fibrotic activity has been demonstrated in an *in vitro* model of hepatic fibrogenesis performed on hepatic stellate cells. The silibinin contained in milk thistle has shown the ability to inhibit growth factor in a dose-dependent manner and consequently decrease collagen production.

The results obtained in the *in vitro* study were confirmed in a model of liver fibrosis induced by excessive alcohol consumption in baboons, in which the administration of silymarin significantly reduced the increase in type I liver collagen induced by excessive alcohol consumption. In recent years, due to the important activities attributed to milk thistle and the important role it could play, given that there is still no pharmacological therapy for the treatment of hepatic steatosis/fibrosis, many clinical studies have been carried out.

The aim of these studies was to evaluate the efficacy of milk thistle and, above all, the effective dose. Most of the studies were conducted as double-blind, placebo-controlled trials, over periods ranging from 3 months to 2 years, on patients with alcoholic and non-alcoholic hepatic steatosis, as well as on patients with cirrhosis and fibrosis. In all these studies, silymarin proved effective in reducing transaminases, improving functional and histological parameters, increasing endogenous antioxidant systems and, finally, increasing life expectancy. However, milk thistle, and in particular its extract known as silymarin, composed of the flavonoids silibin (also called silibinin), isosilibin, silidianin and silicristin, has less than ideal chemical and physical characteristics, which heavily influence its pharmacokinetics.

There are four main causes of the extract's low bioavailability.

The first is its poor solubility in water (0.04 mg/ml), the second is its poor ability to permeate through intestinal epithelial cells, the third is that the already small amount absorbed is extensively metabolised in the liver, and finally, the fourth and last cause is related to its rapid excretion in bile and urine, resulting in equally rapid elimination.

For this reason, various studies have been carried out to evaluate and determine its chemical and physical characteristics, in order to develop formulation strategies that would improve its activity.

In general, the bioavailability of milk thistle can be quantified between 20 and 50%, with peak plasma concentration reached in 2-4 hours and a half-life of around 6 hours.

Once absorbed, milk thistle undergoes rapid phase 1 and phase 2 biotransformation in the liver and is mainly involved in glucuronidation and sulfation reactions that lead to the formation of silibin monoglucuronide, silibin diglucuronide, silibin monosulfate, and silibin diglucuronide sulfate.

The elimination of silymarin, which is the substance to which the greatest activity is attributed, occurs rapidly in both its natural and conjugated forms. Studies conducted on humans suggest that biliary excretion of the conjugated forms is the main route of elimination. To overcome these problems, various methods have been developed to improve certain characteristics in order to achieve greater absorption, better stability and, consequently, allow lower doses to be used.

All data collected to date show that milk thistle, and more specifically silymarin, has significant protective effects on the liver, especially when included in formulations that improve its physical and chemical characteristics. For this reason, it has been hypothesised that a technologically innovative formulation containing milk thistle extract combined with starch, in particular modified starch, chitosan, Phosphatidylserine and phosphatidylcholine may promote stability and absorption, reduce metabolism and, ultimately, lead to improved therapeutic efficacy. According to the inventors, the combination of milk thistle extract with the phospholipids phosphatidylserine and phosphatidylcholine, increases the absorption of the latter, as it improves the ability to cross the epithelial cells of the gastrointestinal tract.

In particular, silymarin is characterised by a poor ability to permeate the epithelial cells of the gastrointestinal tract, which prevents its absorption. Consequently, the addition of phosphatidylserine and phosphatidylcholine would significantly improve absorption.

However, the association of silymarin with phospholipids after oral administration could be degraded by the acidity and digestive enzymes present in the stomach. For this reason, a coating has been proposed that guarantees gastroprotection and controlled release. This dual action can be achieved by using modified starch, which performs two functions in particular.

The first is to protect the phospholipid-silymarin combination from the gastric environment and allow it to reach the intestine intact. the second, no less important than the first, is to achieve a prolonged release in order to reduce hepatic metabolism, which would normally massively counteract traditional silymarin, and a stable plasma concentration over time with consequent greater therapeutic efficacy.

The addition of chitosan also increases the bioavailability of silymarin and its absorption thanks to its mucoadhesive properties, which enable it to bind to the mucins expressed on the cells of the intestinal mucosa, promoting the absorption of the active ingredients it carries. In addition, this molecule appears to be capable of interacting with the *tight junctions* present between the epithelial cells of the intestinal mucosa, facilitating the passage of active ingredients into the systemic circulation.

The present invention therefore aims to provide a nutraceutical or pharmaceutical composition suitable for delivering milk thistle extract to improve efficacy, safety and compliance in patients suffering from hepatobiliary diseases.

The invention also includes a pharmaceutical product or food supplement comprising the nutraceutical or pharmaceutical composition according to the invention. In addition to the active ingredient, namely milk thistle extract, and functional excipients such as starch, chitosan, phosphatidylcholine and phosphatidylserine, the pharmaceutical product or food supplement of the invention may optionally comprise additional active ingredients and functional excipients, which are easily selected by the expert in the field according to the needs of the case. The choice of vehicles, excipients and/or diluents necessary for formulating the pharmaceutical product or food supplement in an appropriate dosage form also falls within the normal capabilities of those skilled in the art.

The nutraceutical or pharmaceutical composition according to the invention is as defined in the attached claim 1.

Further features and advantages of the invention are defined in the dependent claims. The claims form an integral part of this description.

A detailed description of some preferred embodiments of the invention is provided below. As indicated, the nutraceutical or pharmaceutical composition of the present invention comprises a milk thistle extract as an active ingredient and a combination of functional excipients that provide the composition with properties of gastroresistance, increased permeability, mucoadhesion, controlled release and increase the bioavailability of the functional substances of the extract (silymarin). This combination of functional excipients includes starch, chitosan, phosphatidylcholine and phosphates-dilserine.

The nutraceutical or pharmaceutical composition of the invention is therefore particularly effective for the delivery and supplementation of milk thistle extract for the prevention and/or treatment of hepatobiliary diseases. Examples of such conditions and diseases are hypertransaminasemia, infectious toxic alcoholic liver disease, acute hepatitis, chronic hepatitis, hepatic steatosis, non-alcoholic hepatic steatosis, hepatic fibrosis, biliary retention, hepatic cirrhosis, and halitosis.

Starch is an organic compound of a polysaccharide nature consisting of repeating glucose units linked by α-glycosidic bonds. It consists of two types of polymers: amylose, which generally amounts to about 20%, and amylopectin, which generally amounts to about 80%. Amylose forms the central part of starch granules, is soluble in very hot water and consists of glucose molecules linked by α-1,4 glycosidic bonds. Amylopectin is a polymer with a high degree of branching that forms the outer part of the granules. The monomeric units that compose it are joined at the branching points by α-1,6 glycosidic bonds. In nature, it forms in the green parts of plants and is then accumulated in storage organs such as tubers, seeds and roots. Due to its properties and characteristics, it has always had numerous industrial uses.

Starch is particularly important in the food industry, which uses it as a thickening agent and in the production of sweeteners such as maltitol and sorbitol. Thanks to its adhesive properties, it is also used in the production of paper and glues, in the form of starch paste. In the pharmaceutical industry, starch has always been used as an excipient and for the formation of coatings, thanks to its binding properties.

Although starch is also used in its natural form, companies are mainly interested in modified starches, i.e. starch molecules that have been suitably modified to meet the requirements of the various production processes in which it is used. These modified starches can be obtained by using plants that have undergone natural or induced genetic mutations and therefore produce starches with altered characteristics. Another strategy is to modify starch, generally derived from corn, tapioca and rice, by means of chemical treatments (addition of functional groups, treatment with acids and bases), physical treatments (gelatinisation) or enzymatic treatments (partial hydrolysis). Dextrins are an example of modified starches obtained by hydrolysis and repolymerisation. These reactions can be carried out either by simple thermal degradation or by acid catalysis. The result is molecules characterised by shorter chains and therefore partially or totally soluble in water. Examples of dextrins are cyclodextrins and maltodextrins, excipients widely used in the nutraceutical and pharmaceutical fields.

The nutraceutical and pharmaceutical industries have shown great interest in modified starches with a high amylose content (HAS, *High Amylose Starch*).

To be defined as such, a starch must have an amylose content of at least 50%. High amylose starch can be obtained from genetically modified plants or by enriching starches with low amylose content with amylose. The strategies employed by various companies involve the use of HAS for the preparation of solid pharmaceutical forms or in coating processes. HAS has several advantages over other types of starch, such as better consistency, greater thermal stability and greater resistance to moisture and adhesion. Various strategies have been implemented to exploit the advantages of HAS in the most appropriate way. Scherer Corporation, for example, is credited with the use of soft gel capsules in which a certain percentage of gelatin is replaced with the aforementioned starch. The capsules obtained in this way have a better appearance and greater resistance. The Dow Chemical Company boasts the use of more uniform capsules with greater stability in water and at high temperatures thanks to the use of hydroxyalkylated HAS. The Upjohn Company boasts the use of amylose acetate phthalate as a coating agent in gastro-resistant preparations.

Various types of starch can be used in this invention. By way of example, we mention: a chemically unmodified pregelatinised corn starch, or an acetylated pregelatinised corn starch with a high amylose content which, in this specific case, can reach up to 90% by weight. The percentage of acetyl groups is between 0.5% and 2.5%.

Acetylation is carried out with acetic anhydride, which guarantees that the percentage of acetyl groups will be greater than 0.5% but not greater than 2.5%. The starch pregelatinisation treatment consists of dispersing the acetylated starch in water and subjecting the resulting dispersion to temperatures between 100 and 130 degrees and high pressures. The starch granules subjected to this procedure explode and form a gel with a moisture content of between 5 and 10%. Once solidified and removed, the modified starch thus obtained can be used in the coating processes of hard, soft and microgranule capsules and guarantees a coating that is both resistant and suitably viscous, capable of masking unpleasant odours and flavours and which can also be used when a gastro-resistant or modified-release pharmaceutical form is desired. The characteristics of the pharmaceutical form can be modulated by changing the amount of starch used in the coating.

Tests conducted using the above-mentioned starch have shown its gastro-resistant action, protection against moisture and ability to release the active ingredient after a few minutes in the intestinal environment.

Another functional excipient covered by this invention is chitosan. This is a polysaccharide molecule obtained by partial deacetylation of the chitin present in the shells of marine crustaceans. Specifically, it is a polymer formed by repeating units of glucosamine and N-acetylglucosamine, held together by β-1,4 glycosidic bonds. This structure can differ in terms of molecular weight (50-200 kDa), viscosity and degree of deacetylation (40-98%). Generally, the term chitosan is used for all polymers with the above structure that have a degree of deacetylation of at least 70%.

This polymer is widely used in the therapeutic field due to its numerous properties. Recent studies focus on the use of chitosan as an excipient in various pharmaceutical forms, including controlled-release tablets and granules, for the delivery of active ingredients. Specifically, chitosan has mucoadhesive properties. In the intestine, more precisely in the colon, which has a slightly acidic pH, the amino groups of chitosan tend to protonate, thus allowing the molecule to acquire a positive charge. In this way, chitosan can establish electrostatic interactions with the mucins expressed on the surface of the epithelial cells of the intestinal mucosa. These mucoadhesive properties of chitosan ensure greater permeability of silymarin throughout the gastrointestinal tract.

Furthermore, an *in vivo* study has shown that chitosan is also able to interact with the *tight junctions* between the epithelial cells of the intestinal mucosa. This effect is amplified by the presence of phospholipids, which facilitate the passage of active substances through cell membranes into the systemic circulation. As indicated, additional functional excipients present in the nutraceutical or pharmaceutical composition of the invention are phosphatidylcholine and phosphatidylserine. These compounds are part of the large class of phospholipids. Phospholipids are molecules that are structurally very similar to triglycerides. They are composed of a glycerol molecule esterified in positions 1 and 2 with fatty acids. The fatty acids that enter into the composition of natural phospholipids can have a length between 12 and 22 carbon atoms; position 1 generally contains a saturated fatty acid, position 2 contains unsaturated fatty acids, and position 3 contains a phosphate group which, in turn, is esterified with a complex molecule of various types, such as choline, serine, ethanolamine or inositol. These molecules give the phospholipid its name (phosphatidylcholine, phosphatidylserine, phosphatidylethanolamine and phosphatidylinositol) and influence its physical properties, as they determine whether the molecule is anionic (such as phosphatidylserine) or zwitterionic (such as phosphatidylcholine).

Their unique chemical structure makes phospholipids amphiphilic molecules, i.e. molecules capable of interacting with both polar and non-polar solvents. More precisely, the carbon chains of fatty acids represent the non-polar portion that interacts with non-polar solvents, while the head of the phospholipid, consisting of the phosphate group and the molecule bound to it, represents the polar part that interacts with water and other polar solvents. This structural characteristic makes them molecules with surfactant properties which, above a certain temperature, defined as the critical micelle concentration, aggregate to form characteristic complexes that can vary in shape and size depending on the conditions of the environment in which they are formed and the length of the fatty acid chains that compose them. For example, when dispersed in aqueous solution, they generate micelles with a typical shape, with the polar heads facing outwards towards the aqueous environment and the hydrophobic tails facing inwards. When dispersed in organic solvents, they form so-called inverse micelles, in which the heads face inwards and the tails face the external non-polar environment. In terms of size, they vary according to the length of the carbon chains of the fatty acids that make up the phospholipids.

Phospholipids are molecules with significant biological importance, firstly because they are part of the composition of biological membranes and, secondly, because they are involved in various complex mechanisms such as intracellular signal transduction, the regulation of the intracellular concentration of certain ions and the mediation of inflammatory processes as sources of arachidonic acid. Phospholipids are also interesting molecules from a technological point of view and are therefore used in the pharmaceutical and nutraceutical fields as technological adjuvants in the formulation of delivery systems for various active ingredients. Many active ingredients may in fact have poor bioavailability due to the difficulty of crossing the biological barriers and membranes of many areas of the body. Phospholipids, and in particular phosphatidylcholine and phosphatidylserine, are a valuable aid in the technological strategies for the release of various active ingredients. One of the advantages of phospholipid-based delivery systems is the compatibility of phospholipids with cell membranes, both at the mucosal and skin levels. Phosphatidylcholine and phosphatidylserine therefore act as *enhancers* of intestinal and topical absorption, and this action can be attributed to the following mechanisms:
- thanks to their properties and structure, they can merge with the lipids of the stratum corneum and membranes and disrupt their structure, allowing various substances to pass through;
- when in contact with intestinal fluids, they form micelles that help increase the absorption of the active ingredients of interest as they extract lipids from the membranes and alter the rheological properties, fluidity and composition of the membranes, increasing permeability;
- These micelles protect the active ingredients from chemical and enzymatic degradation and can be absorbed into the enterohepatic circulation of bile salts, together with mixed micelles from the diet, and transported into the bloodstream where they release the incorporated active ingredients.

The present invention therefore makes it possible to obtain:
- Gastric resistance;
- Increased permeability through biological membranes and tight junctions;
- Mucosal adhesion;
- Controlled release of silymarin;
- Increased bioavailability of silymarin. The present invention constitutes a ready and effective intervention useful for the prevention and/or treatment of hepatobiliary diseases. This effect is attributed to the combined action of the substances that constitute it. The starch used in the present invention, It masks the unpleasant taste that can be found in supplements based on natural extracts, as well as protecting the functional substances of milk thistle from the acidic pH of the stomach and enzymatic degradation, ensuring their controlled release in the intestine. The chitosan used in the present invention, promotes greater absorption of silymarin, increasing its contact time with the cells of the intestinal mucosa through the establishment of electrostatic interactions and its passage into the systemic circulation through cell membranes, interacting with the tight junctions present on epithelial cells.

Phosphatidylcholine and phosphatidylserine increase the bioavailability of silymarin thanks to their multi-mechanism action on the intestinal mucosa.

The efficacy of the nutraceutical or pharmaceutical composition that is the subject of this invention is evaluated using the experimental protocol described below.

In order to evaluate gastroresistance, a disintegration test is performed, as prescribed by the Pharmacopoeia.

A sample of the pharmaceutical form to be tested is placed in a special instrument containing 0.1 N hydrochloric acid. The gastroresistant pharmaceutical form, when in contact with the buffer at pH 2 for two hours, does not undergo disintegration. The sample is then transferred to a buffer at pH 6.8, in which it disintegrates within ten minutes.

Gastro-resistance can also be assessed using a dissolution test from the Pharmacopoeia, according to which the pharmaceutical form is placed in contact with a 0.1 N hydrochloric acid solution and, in order to pass the test, must release less than 20% of silymarin after 2 hours.

To evaluate intestinal permeability, an *in vitro* test is performed on Caco-2 cells. The cells are cultured using a suitable growth medium (e.g. containing FBS, foetal bovine serum) and kept under controlled conditions (e.g. at 37°C, in an atmosphere of 5% CO₂ and 100% humidity). After several steps, the cells are washed and preincubated with PBS. Following treatment with the formulations of interest, the samples taken are analysed to quantify silymarin.

The efficacy of the present invention can be evaluated by monitoring the antioxidant activity of the formulated silymarin compared to silymarin as such using methods known to those skilled in the art (DPPH, FRAP, ABTS, ORAC, or other suitable tests) in *in vitro* or *in vivo* models. The nutraceutical or pharmaceutical composition of the present invention is particularly effective in the prevention and/or treatment of hepatobiliary pathologies thanks to the synergistic action of its components.

As indicated above, the nutraceutical or pharmaceutical composition of the present invention is incorporated into a pharmaceutical product or food supplement, which is formulated in a suitable dosage form, the composition and preparation of which is within the capabilities of a person skilled in the art.

In a preferred embodiment, the milk thistle extract in the nutraceutical or pharmaceutical composition of the invention is present in an amount between 0.1 and 90%, preferably between 0.5 and 80%, and even more preferably between 1 and 70%, relative to the total weight of the composition.

By way of example, further percentages of milk thistle extract that can be used in the composition of the invention are: 2%, 3%, 4%, 6%, 7%, 8%, 9%, 10%, 15%, 20%, 25%, 30%, 35%, 40%, 45%, 50%, 55%, 60%, 65%, 75%, or 85%.

In another preferred embodiment, the starch in the nutraceutical or pharmaceutical composition of the invention is present in an amount between 0.1 and 90%, preferably between 0.5 and 80%, and even more preferably between 1 and 70% relative to the total weight of the composition.

By way of example, further percentages of starch that can be used in the composition of the invention are: 2%, 3%, 4%, 6%, 7%, 8%, 9%, 10%, 15%, 20%, 25%, 30%, 35%, 40%, 45%, 50%, 55%, 60%, 65%, 75%, or 85%.

In still another preferred embodiment, the chitosan in the nutraceutical or pharmaceutical composition of the invention is present in an amount between 1 and 10%, preferably between 0.5 and 8%, and even more preferably between 0.1 and 5% relative to the total weight of the composition.

By way of example, further percentages of chitosan that can be used in the composition of the invention are: 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5%, 5%, 6%, 7%, 8%, 9%, 10%.

In yet another preferred embodiment, the phosphatidylcholine in the nutraceutical or pharmaceutical composition of the invention is present in an amount between 0.01 and 50%, preferably between 0.05 and 30%, and even more preferably between 0.1 and 10% relative to the total weight of the composition.

By way of example, further percentages of phosphatidylcholine that can be used in the composition of the invention are: 0.02%, 0.03%, 0.04%, 0.06%, 0.07%, 0.08%, 0.09%, 0.2%, 0.3%, 0.4%, 0.6%, 0.7%, 0.8%, 0.9%, 1%, 2%, 3%, 4%, 5%, 6%, 7%, 8%, 9%, 15%, 20%, 25%, 35%, 40%, or 45%.

In yet another preferred embodiment, phosphatidylserine in the nutraceutical or pharmaceutical composition of the invention is present in an amount between 0.01 and 50%, preferably between 0.05 and 30%, more preferably between 0.1 and 10% relative to the total weight of the composition.

By way of example, further percentages of phosphatidylserine that can be used in the composition of the invention are: 0.02%, 0.03%, 0.04%, 0.06%, 0.07%, 0.08%, 0.09%, 0.2%, 0.3%, 0.4%, 0.6%, 0.7%, 0.8%, 0.9%, 1%, 2%, 3%, 4%, 5%, 6%, 7%, 8%, 9%, 15%, 20%, 25%, 35%, 40%, or 45%.

All of the above preferred embodiments can be combined with each other.

The pharmaceutical product or food supplement, which comprises the pharmaceutical or nutraceutical composition of the invention, is formulated in a preferably oral pharmaceutical form, which may be solid, semi-solid or liquid.

Examples include a powder, a buccal powder, a granulate, a hard capsule, a soft-gel capsule, a tablet, a sachet, a solution, a suspension or an emulsion.

Below are some non-limiting examples of nutraceutical or pharmaceutical compositions that are the subject of this invention. As indicated above, these nutraceutical or pharmaceutical compositions are formulated as pharmaceutical products or food supplements and administered in a suitable oral dosage form, possibly divided into one or more dosage units, such as, for example, a capsule, a tablet or a sachet.

The following examples are provided for illustrative purposes only and are not intended to limit the scope of the invention as defined by the appended claims.

### EXAMPLES

### EXAMPLE 1

| ***Active principle*** | ***Quantity %*** |
|---|---|
| Milk thistle extract | 54 |
| MODIFIED STARCH AMPRAC01 44.2 acetylated | 41, 9 |
| Phosphatidylcholine 30% | 2,4 |
| Phosphatidylserine 60 % | 1,2 |
| Chitosan | 0,5 |

### EXAMPLE 2

| ***Active principle*** | ***Quantity* %** |
|---|---|
| Cardo mariano estratto | 54 |
| PREGELATINISED CORN STARCH VN 44.2 | 43,2 |
| Phosphatidylcholine 30% | 1, 2 |
| Phosphatidylserine 60 % | 0,6 |
| Chitosan | 1 |

### EXAMPLE 3

| ***Active principle*** | ***Quantity %*** |
|---|---|
| Milk thistle extract | 54 |
| MODIFIED STARCH AMPRAC01 44.2 acetylated | 42,7 |
| Phosphatidylcholine 30% | 1,2 |
| phosphatidylserine 60 % | 0, 6 |
| Chitosan | 1,5 |

### EXAMPLE 4

| ***Active principle*** | ***Quantity %*** |
|---|---|
| Milk thistle extract | 40 |
| PREGELATINISED CORN STARCH VN 44.2 | 53 |
| Phosphatidylcholine 30% | 3, 6 |
| phosphatidylserine 60 % | 1, 4 |
| Chitosan | 2 |

### EXAMPLE 5

| ***Active principle*** | ***Quantity %*** |
|---|---|
| Milk thistle extract | 60 |
| MODIFIED STARCH AMPRAC01 44.2 acetylated | 34,9 |
| Phosphatidylcholine 30% | 2,5 |
| phosphatidylserine 60 % | 2, 5 |
| Chitosan | 0, 1 |

### EXAMPLE 6

For the preparation of the product 'granular milk thistle', fluid bed granulation technology can be used, for example. Below is an example of preparation, applied to the compositions of the Examples.

The manufacturing process consists of the following stages:
a) Mixing:
   The raw materials previously loaded into the granulator basket undergo an initial mixing stage in a fluidised bed, with process air at a specific temperature (e.g. 80-90°C), until a mixture with an average temperature of approximately 44°C is obtained. During this phase, a homogeneous bulk is created in terms of composition and temperature, which is an essential prerequisite for the optimal progress of the subsequent granulation phase.
b) Granulation
   The granulation phase involves the addition of an aqueous solution of a suitably chosen binding or granulation agent, by means of direct nebulisation onto the premixed bulk and fluidised bed. Process air at 90°C, for example, is also used in this phase, selecting the appropriate speed of introduction of the binding solution to obtain a granular product that is structured as expected (grain size, bulk density, flowability) and homogeneous.
c) Drying
   During the drying phase, the water content of the preformed granules is brought back to the conditions of the mixture of the starting raw materials. The temperature of the process air of the granules at the end of the phase is appropriately evaluated during pilot tests in relation to this objective.
d) Calibration
   The semi-finished product obtained from the previous phase is transferred from the fluidised bed granulator to an oscillating granulator where it is calibrated through a sieve to reduce the particle size of the granules and agglomerates with a coarser structure.

## Claims

1. A nutraceutical or pharmaceutical composition comprising the combination of an extract of *Silybum marianum,* phosphatidylcholine, phosphatidylserine, starch and chitosan.

2. The nutraceutical or pharmaceutical composition according to claim 1 for use in the treatment or prevention of hepatobiliary conditions or diseases in both humans and animals.

3. The nutraceutical or pharmaceutical composition for use according to claim 1 or 2, comprising an extract of *Silybum marianum* comprised between 0,1 and 90%, preferably between 0.5 and 80%, even more preferably between 1 and 70% with respect to the total weight of said combination

4. The nutraceutical or pharmaceutical composition for use according to any one of claims 1 to 3, comprising starch, preferably starch, preferably pregelatinised acetylated starch, preferably in an amount of between 0.1 and 90%, more preferably between 0.5 and 80%, and even more preferably between 1 and 70% with respect to the total weight of said combination.

5. The nutraceutical or pharmaceutical composition for use according to any one of claims 1 to 4, comprising an amount of phosphatidylcholine comprised between 0,01 and 50%, preferably between 0,05 and 30%, more preferably between 0,1 e 10% with respect to the total weight of said combination and an amount of phosphatidylserine comprised between 0,01 and 50%, preferably between 0,05 e 30%, more preferably between 0,1 e 10% with respect to the total weight of said combination.

6. The nutraceutical or pharmaceutical composition for use according to any one of claims 1 to 5, comprising chitosan preferably in an amount of between 1 and 10%, more preferably between 0.5 and 8%, even more preferably between 0.1 e 5% with respect to the total weight of said combination.

7. The nutraceutical or pharmaceutical composition for use according to any one of claims 1 to 6, wherein the hepatobiliary condition or disease is selected from the group consisting of hypertransaminasemia, infectious toxic alcoholic liver disease, acute hepatitis, chronic hepatitis, hepatic steatosis, non-alcoholic hepatic steatosis, hepatic fibrosis, bile retention, hepatic cirrhosis, halitosis.

8. A Pharmaceutical product or food supplement according to claim 7, formulated in a liquid, semi-solid or solid oral dosage form.

9. The pharmaceutical product or food supplement according to claim 7, wherein in cui the oral dosage form is a powder, an orodispesible powder, granules, a hard capsule, a soft-gel capsule, a tablet, a sachet, a solution, a suspension or an emulsion.
